# EUROPEAN PATENT APPLICATION

(11) **EP 4 691 495 A1**
(43) Date of publication of application: **11.02.2026**
(21) Application number: 24778191.7
(22) Date of filing: 29.03.2024
(51) Int. Cl.: A61K 47/68, A61K 39/395, A61P 35/00

(54) **USE OF DRUG COMBINATION CONTAINING ANTI-CD20 ANTIBODY-DRUG CONJUGATE IN PREPARATION OF DRUG FOR TREATING NHL**

(30) Priority: 31.03.2023 CN 202310337027
(71) Applicant: Zhejiang Teruisi Pharmaceutical Inc., Huzhou, Zhejiang 313000 (CN)
(72) Inventor: HUANG, Kai, Huzhou, Zhejiang 313000 (CN); LIU, Shicheng, Huzhou, Zhejiang 313000 (CN); ZHANG, Jian, Huzhou, Zhejiang 313000 (CN); HUANG, Jingyao, Huzhou, Zhejiang 313000 (CN); WANG, Chen, Huzhou, Zhejiang 313000 (CN); SHI, Mingbiao, Huzhou, Zhejiang 313000 (CN); YU, Xiao, Huzhou, Zhejiang 313000 (CN); HAN, Ying, Huzhou, Zhejiang 313000 (CN)
(74) Representative: Haseltine Lake Kempner LLP
(86) International application number: PCT/CN2024/084657
(87) International publication number: WO 2024/199405

(57) **Abstract**

The present disclosure discloses a use of a drug combination containing an anti-CD20 antibody drug conjugate (ADC) in the preparation of a drug for treating Non-Hodgkin Lymphoma (NHL). The drug combination includes the anti-CD20 ADC and at least one therapeutic agent. The drug combination therapy has better efficacy than the existing clinical first-line standard therapy. Additionally, it offers several advantages, including fewer types of drugs, shorter infusion time, lower safety risks, and better economic accessibility.

## Description

### CROSS-REFERENCE TO RELATED APPLICATIONS

This application claims the benefit of priority to the China Inventio n Patent Application No. CN202310337027.9 filed on March 31, 20 23, the entire contents of the aforementioned application are incorpo rated herein by reference.

### TECHNICAL FIELD

The present disclosure relates to the field of pharmaceutical and dru g combination technology, and particularly relates to the use of a d rug combination containing an anti-CD20 antibody drug conjugate (ADC) in the preparation of a drug for treating Non-Hodgkin Lymp homa (NHL).

### BACKGROUND

Non-Hodgkin Lymphoma (NHL) is the seventh most common type of cancer in the world, accounting for ~4% to 5% of new cancer cases and 3% to 4% of cancer-related deaths, affecting ~1.5 million people worldwide. NHL is a general term for a group of malignant proliferative diseases of the lymphatic system, including many types of lymphocytomas, which mainly originate from B lymphocytes (> 85%), and a few originate from T cells and natural killer cells. It is usually classified as indolent NHL (iNHL) and aggressive NHL (aNHL) according to the prognosis. Among them, follicular lymphoma (FL) is the most common iNHL, accounting for ~1/5 of all NHL patients, while diffuse large B-cell lymphoma (DLBCL) is the most common aNHL, accounting for ~1/3 of all NHL patients.

In the revised 2016 World Health Organization (WHO) Classification of Lymphoid Neoplasms, double expressor lymphoma (DEL) is defined as overexpression of MYC and BCL-2 proteins not related to chromosomal rearrangements. DEL accounts for 20% to 30% of newly diagnosed DLBCL, and is a group of highly aggressive lymphomas with progression-free survival (PFS).

The first-line treatment regimen recommended by clinical guidelines for most NHL subtypes containing DLBCL includes a quadruple therapy: Rituximab (R) combined with Cyclophosphamide (C) + Doxorubicin (H) + Vincristine (O) + Prednisone (P) (R-CHOP). Although most NHL subtype patients with R-CHOP therapy have a good response rate, there is still room for further improvement. In addition, this therapy involves a combination of 5 drugs, requires lengthy administration, carries certain infusion-related risks, and incurs high costs.

The newly approved first-line regimen for treating DLBCL is Pola-R-CHP (i.e., replacing the chemotherapy drug Vincristine (O) in the R-CHOP regimen with CD79b-ADC), which does not reduce the number of combined drugs, but only replaces the lower-cost chemotherapy drug O with a more expensive ADC, Polatuzumab (POLIVY). In addition, current clinical trials for first-line treatment of DLBCL include a variety of R-CHOP + X treatment regimens to explore the possibility of improving efficacy based on R-CHOP. However, these treatment approaches primarily build upon R-CHOP by adding more medications, which not only increase the risk of adverse effects for patients but also raise the overall cost of treatment.

Therefore, there remains an urgent need to identify new, more effective therapies or combination regimens for NHL, particularly DLBCL, preferably ones that reduce the number of medications involved while delivering improved outcomes compared to the standard treatment.

### SUMMARY

The purpose of the present disclosure is to provide a drug combination and a use thereof in preparing a drug for treating Non-Hodgkin Lymphoma (NHL). The drug combination therapy has better efficacy than the existing clinical first-line standard therapy, fewer types of medication, shorter infusion time, lower safety risk, and better economic affordability.

**A first aspect of the present disclosure provides a use of an anti-CD20 antibody drug conjugate (ADC) alone or in combination with at least one therapeutic agent in the preparation of a drug for treating NHL.**

The anti-CD20 ADC has the following structure:
mAb-(L-D)n;
Wherein mAb represents a recombinant anti-CD20 monoclonal antibody (mAb), D represents a small molecule toxin; L is a linker connecting the mAb and the small molecule toxin; n is the mean of small molecule toxins coupled to the mAb; and "-" is a bond.

Preferably, the recombinant anti-CD20 mAb includes but is not limited to Rituximab or a biosimilar thereof, Ofatumumab or a biosimilar thereof, Obinutuzumab or a biosimilar thereof, or Ocrelizumab or a biosimilar thereof.

More preferably, the recombinant anti-CD20 mAb is preferred to Rituximab or a biosimilar thereof.

Preferably, D is one or more monomethyl auristatins (MMAs), including but not limited to MMAE, MMAD, or MMAF; further, the MMA is MMAE.

L is a shearable joint or a non-shearable joint. The non-shearable joint includes but is not limited to a thioether bond joint comprising a maleimide group; preferably, the maleimide group comprises hexanoic acid to form maleimidocaproyl (mc); preferably, the maleimide group comprises a maleimidomethyl group, such as succinimidyl 4-(N-maleimidomethyl)cyclohexane-1-carboxylate (sMCC) or sulfo-sMCC.

The shearable joint includes but is not limited to a hydrazone bond joint, a disulfide bond joint, and a peptide bond joint; the peptide bond joint comprises valine-citrulline (Val-Cit or VC), phenylalanine-lysine (Phe-Lys), valine-alanine (Val-Ala), or valine-lysine (Val-Lys). Preferably, the peptide bond joint is Val-Cit.

Further, the joint also contains para-aminobenzyl alcohol (PAB), para-aminobenzyl carbonate (PABC), or a derivative or analog thereof.

Further, L is MC-VC-PAB.

Then is an integer or non-integer from 1 to 8; preferably, n is 4.2 ± 1, more preferably, 4.2 ± 0.5, and still more preferably, 4.2 ± 0.3.

The anti-CD20 ADC further has the following structure:

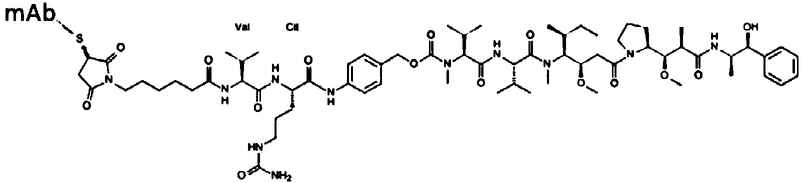

Wherein mAb is Rituximab or a biosimilar thereof, n is the mean number of small molecule toxins coupled to the mAb and is 1 to 8; preferably, n is 4.2 ± 1, further preferably, n is 4.2 ± 0.5, and still more preferably, n is 4.2 ± 0.3.

The anti-CD20 ADC described in the present disclosure can be a pharmaceutical preparation containing an anti-CD20 ADC and a carrier or excipient, and the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is sucrose.

The osmotic pressure regulator includes mannitol or sodium chloride; preferably, the osmotic pressure regulator is mannitol.

The solubilizer includes polysorbate 80, polysorbate 20, orpoloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80.

The at least one therapeutic agent is selected from one or more of an alkylating agent, an antibiotic drug, a botanical drug, a hormone drug, a platinum drug, and an antimetabolite;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin;
the botanical drug is selected from one or more of Vinca Alkaloid, Vinblastine, Vincristine, Vindesine, Vinorelbine, Docetaxel, Camptothecin, Irinotecan, Exatecan, Topotecan, Paclitaxel, and Taxane;
the hormone drug is selected from one or more of Prednisone and Dexamethasone;
the platinum drug is selected from one or more of Carboplatin, Cisplatin, and Oxaliplatin; and
the antimetabolite is selected from one or more of Pemetrexed, Methotrexate, Fluorouracil, Capecitabine, Cytarabine, Gemcitabine, 6-Mercaptopurine, and Tioguanine.

Preferably, the at least one therapeutic agent is an alkylating agent, an antibiotic drug, and a hormone drug;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin;
the hormone drug is selected from one or more of Prednisone and Dexamethasone; and
Further, the at least one therapeutic agent is Cyclophosphamide, Doxorubicin, and Prednisone, that is, the drug combination is the anti-CD20 ADC + Cyclophosphamide + Doxorubicin + Prednisone.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL) , T-cell Large granular lymphocytic leukemia(T-LGLL), or anaplastic large cell lymphoma (ALCL).

Further, the NHL is DLBCL; preferably, the DLBCL is CD20-positive DLBCL.

Further, the DLBCL is newly diagnosed DLBCL; preferably, the newly diagnosed DLBCL is newly diagnosed CD20-positive DLBCL.

Further, the CD20-positive DLBCL is DLBCL with high or low CD20 expression.

Furthermore, the newly diagnosed CD20-positive DLBCL is newly diagnosed DLBCL with high or low CD20 expression.

Furthermore, the CD20-positive DLBCL is DLBCL with dual expression of MYC and BCL2, and high or low CD20 expression.

Furthermore, the newly diagnosed CD20-positive DLBCL is newly diagnosed DLBCL with dual expression of MYC and BCL2, and high or low CD20 expression.

Preferably, the present disclosure discloses the use of the combination (anti-CD20 ADC + Cyclophosphamide + Doxorubicin + Prednisone) in the preparation of a drug for treating CD20-positive DLBCL.

Further, the present disclosure discloses the use of the combination (anti-CD20 ADC + Cyclophosphamide + Doxorubicin + Prednisone) in the preparation of a drug for the treatment of newly diagnosedCD20-positive DLBCL.

Further, the present disclosure discloses the use of the combination (anti-CD20 ADC + Cyclophosphamide + Doxorubicin + Prednisone) in the preparation of a drug for treating CD20-positive DLBCL with high or low CD20 expression.

Furthermore, the present disclosure discloses the use of the combination (anti-CD20 ADC + Cyclophosphamide + Doxorubicin + Prednisone) in the preparation of a drug for the treatment of newly diagnosed CD20-positive DLBCL with high or low CD20 expression.

**A second aspect of the present disclosure provides a drug product for treating NHL.**

The drug product comprises an anti-CD20 ADC and at least one therapeutic agent. The anti-CD20 ADC has a structure as described in the first aspect of the present disclosure, and the at least one therapeutic agent has a type as described in the first aspect of the present disclosure.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL ) , T-cell Large granular lymphocytic leukemia(T-LGLL), or anaplastic large cell lymphoma (ALCL).

Further, the NHL is DLBCL; preferably, the DLBCL is CD20-positive DLBCL.

Further, the DLBCL is newly diagnosed DLBCL; preferably, the newly diagnosed DLBCL is newly diagnosed CD20-positive DLBCL.

Further, the CD20-positive DLBCL is DLBCL with high or low CD20 expression.

Furthermore, the newly diagnosed CD20-positive DLBCL is newly diagnosed DLBCL with high or low CD20 expression.

Furthermore, the CD20-positive DLBCL is DLBCL with dual expression of MYC and BCL2, and high or low CD20 expression.

Furthermore, the newly diagnosed CD20-positive DLBCL is newly diagnosed DLBCL with dual expression of MYC and BCL2, and high or low CD20 expression.

Preferably, the drug product for treating NHL comprises the anti-CD20 ADC, Cyclophosphamide, Doxorubicin, and Prednisone.

**A third aspect of the present disclosure provides a treatment method for NHL.**

The treatment method described in the present disclosure comprises administering an effective dose of the anti-CD20 ADC or the drug product disclosed in the second aspect of the present disclosure to a subject for treating NHL, wherein the anti-CD20 ADC in the drug product can be administered before, simultaneously with, and/or after the administration of the at least one therapeutic agent.

Preferably, the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL ) , T-cell Large granular lymphocytic leukemia(T-LGLL), or anaplastic large cell lymphoma (ALCL).

Further, the NHL is DLBCL; preferably, the DLBCL is CD20-positive DLBCL.

Further, the DLBCL is newly diagnosed DLBCL; preferably, the newly diagnosed DLBCL is newly diagnosed CD20-positive DLBCL.

Further, the CD20-positive DLBCL is DLBCL with high or low CD20 expression.

Furthermore, the newly diagnosed CD20-positive DLBCL is newly diagnosed DLBCL with high or low CD20 expression.

Furthermore, the CD20-positive DLBCL is DLBCL with dual expression of MYC and BCL2, and high or low CD20 expression.

Furthermore, the newly diagnosed CD20-positive DLBCL is newly diagnosed DLBCL with dual expression of MYC and BCL2, and high or low CD20 expression.

Preferably, the combination therapy described in the present disclosure comprises the combined administration of an effective dose of an anti-CD20 ADC, Cyclophosphamide, Doxorubicin, and Prednisone.

The combined drug of the present disclosure can be administered by any suitable means, including gastrointestinal, parenteral, pulmonary, and intranasal. Parenteral infusion includes intramuscular, intravenous, intraarterial, intraperitoneal, or subcutaneous administration.

For the prevention or treatment of diseases, a generally suitable method for the anti-CD20 ADC and at least one therapeutic agent of the present disclosure is to administer the drug to a patient in a single or multiple doses. Depending on the type and severity of the disease, the initial candidate dose of the anti-CD20 ADC administered to the patient can be ~1 µg/kg to 100 mg/kg, such as 0.1 mg/kg to 20 mg/kg. For repeated administrations lasting several days or longer, depending on the situation, treatment is usually continued until the desired suppression of disease symptoms occurs. An exemplary dose range of the ADC may be ~0.05 mg/kg to ~10 mg/kg.

The anti-CD20 ADC of the present disclosure is administered to a patient multiple times, and the administration frequency can be once every 1 to 6 weeks, for example, Q2W, Q3W, or Q4W; and most preferably, Q3W. The anti-CD20 ADC and the at least one therapeutic agent are administered to the subject until recovered, progressive disease (PD), or death.

**A fourth aspect of the present disclosure provides a method for non-therapeutic inhibition of NHL cells.**

The method comprises adding an effective dose of the drug product as described in the second aspect of the present disclosure to the NHL cell system.

Preferably, the method for non-therapeutic inhibition of NHL cells in the present disclosure comprises adding an effective dose of the anti-CD20 ADC, Cyclophosphamide, Doxorubicin, and Prednisone to the NHL cell system.

It is to be understood that one, some, or all of the properties of the various embodiments described herein may be combined to form other embodiments of the present disclosure. Specific embodiments of the present disclosure are further described in details as follows.

### BRIEF DESCRIPTION OF DRAWINGS

Embodiments of the present disclosure are described in detail below with reference to the attached figures, wherein:
Figure 1: Tumors Removed from Mice in Each Treatment Group after Sacrifice on Day 35 in WSU-DLCL2 Cell Xenograft Mouse Model;
Figure 2: Tumor Volume Change Trend of Mice in Each Treatment Group in WSU-DLCL2 Cell Xenograft Mouse Model;
Figure 3: Change Trend of Tumor Growth Inhibition Rate of Mice in Each Treatment Group in WSU-DLCL2 Cell Xenograft Mouse Model; and
Figure 4: Tumor Volume Change Trend of Mice in Each Treatment Group in OCI-LY19 Cell Xenograft Mouse Model.

### DESCRIPTION OF EMBODIMENTS

The present disclosure is described below with reference to specific embodiments. It will be appreciated by those skilled in the art that these embodiments are for illustration of the disclosure only and are not intended to limit the scope of the present disclosure in any way.

The experimental methods in the following embodiments are conventional methods unless otherwise specified. The raw materials, reagent materials, and the like used in the embodiments described below are commercially available products unless otherwise specified.

### Definition:

"Rituximab" refers to a monoclonal antibody drug targeting CD20 developed by Roche and approved for marketing by the National Medical Products Administration (NMPA) under the trade name of "MabThera". Rituximab is the generic name, and only the innovator drug can be called Rituximab.

"Rituximab biosimilar" refers to the monoclonal antibody drug developed by other companies in addition to the innovator drug of Rituximab, with identical amino acid sequences and comparable physical and chemical properties, efficacy, pharmacokinetics, and safety to the innovator drug.

"High CD20 expression" refers to cases where the test results for CD20 expression levels in tumor tissues or cells exceed a certain threshold or are relatively high based on conventional evaluation criteria. For instance, high CD20 expression may be identified when immunohistochemical staining indicates strong positivity (+++) and/or positivity (++), as determined by the proportion of stained cells and staining intensity.

"Low CD20 expression" refers to cases where the test results for CD20 expression levels in tumor tissues or cells are below a certain threshold or are relatively low based on conventional evaluation criteria. For instance, low CD20 expression may be identified when immunohistochemical staining indicates weak positivity (+), as determined by the proportion of stained cells and staining intensity.

"Effective dose" refers to the amount that effectively achieves the desired treatment result at the required dose and time period. The effective dose may vary depending on factors such as the individual's disease state, age, gender, and weight, and the ability of the therapeutic agent or combination of therapeutic agents to induce a desired response in the individual.

Although the present disclosure has been generally described, embodiments of the present disclosure will be further disclosed in the following embodiments, which should not be construed as limiting the scope of the claims.

### Embodiment 1: Preparation of Anti-CD20 ADC (TRS005)

An exemplary preparation method of an anti-CD20 ADC (TRS005) is specifically shown in Patent Application CN108452318A, the entire contents of which are incorporated in the present disclosure.

The structure of TRS005 is as follows: 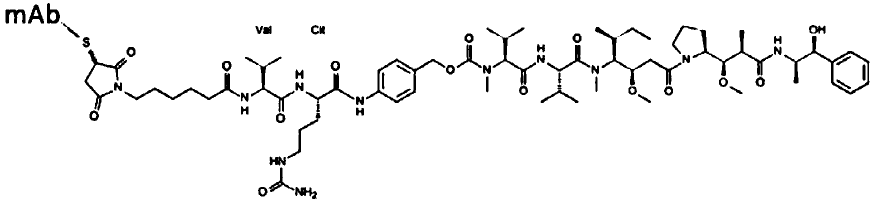 wherein mAb is Rituximab or a biosimilar thereof, and the drug antibody ratio (DAR) is 4.2 ± 0.5.

### Embodiment 2: Screening of Human Lymphoma Cell Lines

By detecting the expression levels (mRNA levels) of BCL-2, MYC, and CD20 in each human DLBCL cell line, the human DLBCL cell line WSU-DLCL2 with high expression of BCL-2, MYC, and CD20 and the human DLBCL cell line OCI-LY19 with high expression ofBCL-2 and MYC and low expression of CD20 were screened out. The expression levels of CD20, BCL2, and MYC in the two cell lines are shown in Table 1.

**Table 1: Expression Levels of CD20, BCL2, and MYC in Two Cell Lines (log₂TPM)**

| | WSU DLCL2 | OCI-LY19 |
|---|---|---|
| CD20 | 9.360825 | 3.690417 |
| BCL2 | 6.178316 | 5.953964 |
| MYC | 7.047015 | 9.049576 |

### Example 3: Inhibition of human lymphoma WSU-DLCL2 cell xenograft tumor growth by TRS005 alone and TRS005 in combination with CHP compared to R-CHOP regimen administration Drug Information, Model Establishment, Grouping, and Administration

The TRS005 finished product is provided by Zhejiang Teruisi Pharmaceutical Inc., and other positive drugs are commercially available. Among them, MabThera, Cyclophosphamide, Doxorubicin, Vincristine, and Prednisone are prepared with normal saline as the solvent to corresponding concentrations and stored at 4°C away from light. The TRS005 finished product is reconstituted with sterile water for injection (WFI), and a corresponding volume of normal saline is added to prepare the corresponding concentration. The TRS005 finished product is reconstituted and packaged with WFI and stored at -70°C.

Under sterile conditions, each SCID mouse was inoculated with 0.1 mL of WSU-DLCL2 cell suspension at a concentration of 4 × 10⁷/mL subcutaneously on the right back. When the mean tumor volume (TV) reached ~150 mm³, animals were randomized into 4 groups according to their TV: Group 1 (Vehicle), Group 2 (R-CHOP), Group 3 (TRS005) and Group 4 (TRS005-CHP (T-CHP)), with 6 mice in each group, so that the difference in TV of mice in each group was < 10% of the mean. The day of grouping was recorded as Day 0, and the administration was started according to animal body weight (BW). The specific grouping and administration regimen are shown in Table 2:

**Table 2 Grouping and Administration Regimen**

| **Grou p** | **Treatment** | **Number of Mice** | **Dose (mg/kg)** | **Drug Concentration (mg/mL)** | **Dose Volume (mL/kg)** | **Route of Administratio n** | **Administration Schedule** |
|---|---|---|---|---|---|---|---|
| 1 | Vehicle | 6 | / | / | 5 | IV | BIW×3w |
| | MabThera (R) | | 10 | 2 | 5 | IV | BIW×3w |
| 2 | Cyclophosphamid e (C) | | 25 | 5 | 5 | IP | QW×3w |
| | Doxorubicin (H) | 6 | 1 | 0.2 | 5 | IV | QW×3w |
| | Vincristine (O) | | 0.5 | 0.1 | 5 | IV | QW×3w |
| 3 | Prednisone (P) | | 0.15 | 0.03 | 5 | PO | QD×5/w×3w |
| | TRS005 | 6 | 0.75 | 0.15 | 5 | IV | BIW×3w |
| 4 | T(TRS005) | 6 | 0.75 | 0.15 | 5 | IV | BIW×3w |
| | Cyclophosphamid e (C) | | 25 | 5 | 5 | IP | QW×3w |
| | Doxorubicin (H) | | 1 | 0.2 | 5 | IV | QW×3w |
| | Prednisone (P) | | 0.15 | 0.03 | 5 | PO | QD×5/w×3w |

| | | | | | | | |
|---|---|---|---|---|---|---|---|
| Wherein, IV: intravenous injection; IP: intraperitoneal injection; PO: oral administration; BIW × 3w: twice a week for three weeks; QW × 3w: once a week for three weeks; QD × 5/w × 3w: once a day for five times a week for three weeks. | | | | | | | |

The BW and TV of animals were measured twice a week during the study. During the study, the clinical symptoms of animals were observed and recorded once a day. Clinical observations included general health status, abnormal BW and behavior of animals, and other treatment-related adverse reactions.

### Evaluation Indicators

The tumor growth inhibition rate (%TGI) is calculated as follows: (1 - TV_{T} / TV_{C}) × 100%, where TV_{C} is the mean TV of the negative control group, and TV_{T} is the mean TV of the treatment group.

Relative Tumor Volume (RTV) is calculated as V_{t /} V₀, where V₀ is the TV at grouping and Vₜ is the TV at each measurement.

The calculation formula for relative tumor proliferation rate (%T/C_{RTV}) is: T_{RTV} / C_{RTV} × 100%, where T_{RTV} is the RTV of the treatment group and C_{RTV} is the RTV of the negative control group.

The animal body weight change (%BWC) is calculated as follows: (BWₜ - BW₀) / BW₀ × 100%, where BWt is the animal BW at each measurement and BW₀ is the animal BW at grouping.

According to the Technical Guidelines for Nonclinical Study of Cytotoxic Anti-tumor Drugs (November 2006) issued by NMPA in China, it is considered effective if %T/C_{RTV} ≤ 40% and P < 0.05 after statistical analysis. Drug doses are considered to be severely toxic when the number of animal deaths related to the drug exceeds 20%.

### Study Results

### (I) Anti-tumor Effect

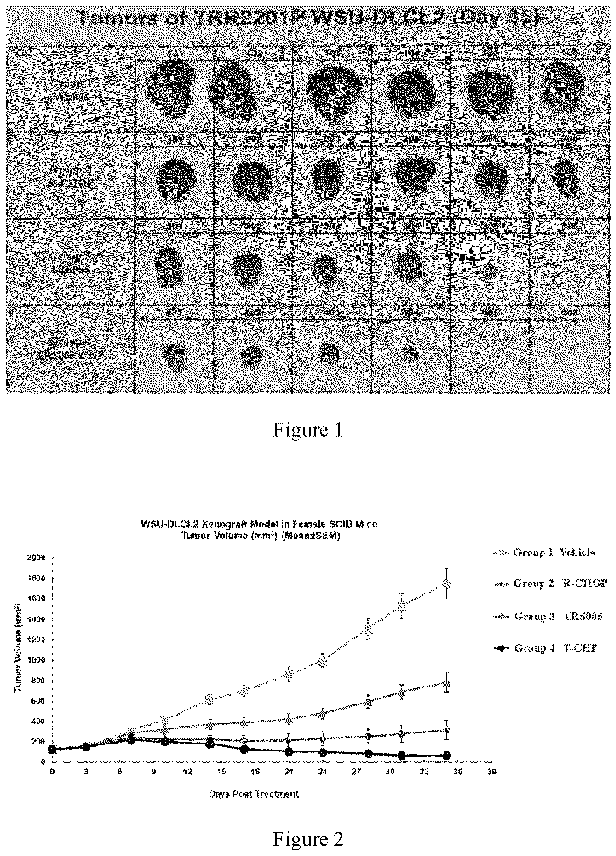

In this study, the effect of TRS005 alone or in combination on the TV of human lymphoma WSU-DLCL2 is shown in Table 3 and Figures 1 to 3.

**Table 3 Effect of Treatment on Tumor Volume in Each Group**

| **Group** | **Number of Mice** | **Tumor Volume (mm³, Mean ± SEM)** | | **%TGI Day 35** | **RTV Day 35** | **%T/C_{RTV} Day 35** |
|---|---|---|---|---|---|---|
| | | **Day 0** | **Day 35** | | | |
| 1 | 6 | 127.51 + 5.95 | 1749.23 + 149.55 | / | 13.70 + 0.94 | / |
| 2 | 6 | 126.80 + 6.53 | 782.94 + 94.87*** | 55.24 | 6.27 + 0.80*** | 45.77 |
| 3 | 6 | 127.16 + 6.60 | 315.93 + 94.23*** | 81.94 | 2.44 + 0.78*** | 17.81 |
| 4 | 6 | 126.99 + 6.25 | 65.73 + 26.35***###$ | 96.24 | 0.55 + 0.22***###$ | 4.01 |

| | | | | | | |
|---|---|---|---|---|---|---|
| **Note: "***" indicates *P* < 0.001 compared to Group 1; "###" indicates *P* < 0.001 compared to Group 2; "$" indicates *P* < 0.05 compared to Group 3.** | | | | | | |

Based on Table 3 and Figures 1 to 3, on Day 35, the mean TV of Group 4 (TRS005-CHP) was 65.73 ± 26.35 mm³, which was significantly lower than 782.94 ± 94.87 mm³ of Group 2 (R-CHOP), *P* < 0.001, and was lower than 315.93 ± 94.23 mm³ of Group 3 (TRS005), *P* < *0.05.*

On Day 35, %TGI of Group 4, Group 3, and Group 2 were 96.24%, 81.94%, and 55.24%, respectively. The above-mentioned results showed that the anti-CD20 ADC (TRS005) of the present disclosure, combined with the CHP regimen, could significantly inhibit the growth of tumor cells with high CD20 expression compared with the R-CHOP regimen and the TRS005 monotherapy regimen, and achieved a significantly improved efficacy. Meanwhile, TRS005 monotherapy achieved better efficacy than the R-CHOP regimen.

### (II) Effect of Test Article on Body Weight of Tumor-bearing Animals

The results of the effect of TRS005 alone or in combination on the BW of WSU-DLCL2 tumor-bearing animals showed that on Day 35, the mean BW of animals in each group increased by 1.67% to 8.39% (0.34 g to 1.68 g) compared with the start of the study on Day 0. The effect of treatment on the BW of tumor-bearing animals in each group is shown in Table 4.

**Table 4 Effect of Treatment on Body Weight of Tumor-bearing Animals in Each Group**

| **Group** | **Number of Mice** | **Body Weight of Mice (g, Mean±SEM)** | | **%BWC Day 35 vs Day 0** | **Number of Mice with Drug-related Deaths Day 35** |
|---|---|---|---|---|---|
| | | **Day 0** | **Day 35** | | |
| 1 | 6 | 20.13 ± 0.37 | 21.81 ± 0.35 | 8.39% ( 1.68 g) | 0/6 |
| 2 | 6 | 20.29 ± 0.42 | 21.04 ± 0.66 | 3.59% ( 0.75 g) | 0/6 |
| 3 | 6 | 20.12 ± 0.35 | 20.78 ± 0.50 | 3.25% ( 0.66 g) | 0/6 |
| 4 | 6 | 20.44 ± 0.51 | 20.78 ± 0.52 | 1.67% ( 0.34 g) | 0/6 |

During the study, the BWC of mice in each treatment group was within the normal range, no drug-related mouse deaths occurred, and no other obvious drug-related toxicity was observed, indicating that the drug combination regimen of TRS005 and CHP and the TRS005 monotherapy regimen of the present disclosure have good safety.

### Embodiment 4: Growth inhibition of human lymphoma OCI-LY19 cell xenograft tumors by TRS005 alone and TRS005 in combination with CHP compared to R-CHOP regimen administration

### Drug Information, Model Establishment, Grouping, and Administration

The drug information is the same as in Embodiment 3. Under sterile conditions, each mouse was inoculated with 0.1 mL of OCI-LY19 cell suspension at a concentration of 2 × 10⁶/mL subcutaneously on the right back. When the mean TV reached ~150 mm³, animals were randomized into 4 groups according to their TV: Group 1 (Vehicle), Group 2 (R-CHOP), Group 3 (TRS005) and Group 4 (T-CHP), with 10 mice in each group, so that the difference in TV of mice in each group was < 10% of the mean. The day of grouping was recorded as Day 0, and the administration was started according to animal BW. The specific grouping and administration regimen are shown in Table 5:

**Table 5 Grouping and Administration Regimen**

| **Group** | **T1 Treatment** | | **Number of Mice** | **Dose (mg/kg)** | **Drug Concentration (mg/mL)** | **Dose Volume (mL/kg)** | **Route of Administration** | **Administration Schedule** |
|---|---|---|---|---|---|---|---|---|
| 1 | Vehicl e | WFI | 10 | / | / | 5 | IV | BIW×3w |
| | | Cyclophosph amide | | 20 | 4 | 5 | IP | QW×3w |
| 2 | R-CHOP | Doxorubicin | 10 | 1 | 0.2 | 5 | IV | QW×3w |
| | | Vincristine | | 0.05 | 0.01 | 5 | IV | QW×3w |
| 3 | TRS0 05 | Prednisone | | 0.1 | 0.02 | 5 | PO | QD×5/w×3w |
| | | MabThera | | 10 | 2 | 5 | IV | BIW×3w |
| | | TRS005 | 10 | 1.5 | 0.3 | 5 | IV | BIW×3w |
| | | Cyclophosph amide | | 20 | 4 | 5 | IP | QW×3w |
| 4 | T-CHP | Doxorubicin | 10 | 1 | 0.2 | 5 | IV | QW×3w |
| | | Prednisone | | 0.1 | 0.02 | 5 | PO | QD×5/w×3w |
| | | TRS005 | | 1.5 | 0.3 | 5 | IV | BIW×3w |

| | | | | | | | | |
|---|---|---|---|---|---|---|---|---|
| Wherein, IV: intravenous injection; IP: intraperitoneal injection; PO: oral administration; BIW × 3w: twice a week for three weeks; QW × 3w: once a week for three weeks; QD × 5/w × 3w: once a day for five times a week for three weeks. | | | | | | | | |

The BW and TV of animals were measured twice a week during the study. During the study, the clinical symptoms of animals were observed and recorded once a day. Clinical observations included general health status, abnormal BW and behavior of animals, and other treatment-related adverse reactions. During the study, if an individual experimental animal meets any of the following conditions, the animal will be removed from the study group and euthanized. 1. The body weight loss (BWL) of animals was ≥ 20% compared with Day 0; 2. Animals had serious adverse reactions, such as blindness and paralysis; 3. TV was > 2000 mm³; 4. Open ulcers were formed on the tumor surface.

### Evaluation Indicators

The calculation formula for TV is: 1 / 2 × a × b², where a and b are the measured length and width of the tumor, respectively.

The tumor growth inhibition rate (%TGI) is calculated as follows: (1 - TV_{T} / TV_{C}) × 100%, where TV_{C} is the mean TV of the negative control group, and TV_{T} is the mean TV of the treatment group.

Relative Tumor Volume (RTV) is calculated as V_{t /} V₀, where V₀ is the TV at grouping and Vₜ is the TV at each measurement.

The calculation formula for relative tumor proliferation rate (%T/C_{RTV}) is: T_{RTV} / C_{RTV} × 100%, where T_{RTV} is the RTV of the treatment group and C_{RTV} is the RTV of the negative control group.

The animal body weight change (%BWC) is calculated as follows: (BWt - BW0) / BW0 × 100%, where BWt is the animal BW at each measurement and BW0 is the animal BW at grouping.

### (I) Anti-tumor Effect

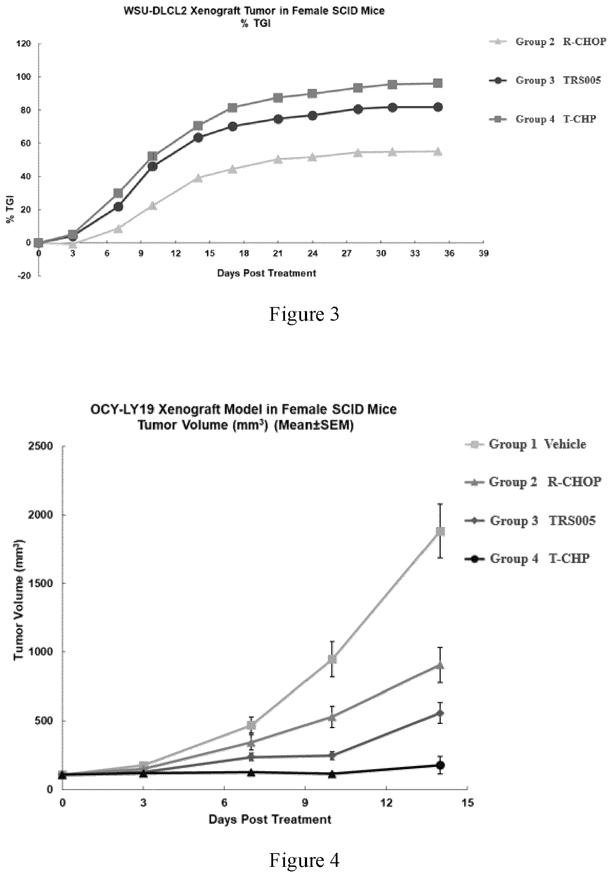

In this study, the effect of TRS005 alone or in combination with CHP on TV of human lymphoma OCI-LY19 is shown in Tables 6 and 7 and Figure 4.

**Table 6 Effect of Treatment on Tumor Volume in Each Group from D0 to D14**

| **Group** | **Number of Mice** | **Tumor Volume (mm³, Mean ± SEM)** | | **%TGI Day 14** | **RTV Day 14** | **%T/C_{RTV} Day 14** |
|---|---|---|---|---|---|---|
| | | **Day 0** | **Day 14** | | | |
| 1 | 10 | 108.73 ± 5.15 | 1881.01 ± 196.11 | / | 17.17 ± 1.56 | / |
| 2 | 10 | 109.54 ± 5.51 | 557.11 ± 76.43 *** | 70.38 | 5.03 ± 0.63 *** | 29.30 |
| 3 | 10 | 107.78 ± 4.71 | 905.30 ± 129.66 *** | 51.87 | 8.29 ± 1.11 *** | 48.28 |
| 4 | 10 | 106.66 ± 4.19 | 176.27 ± 63.80 ***##$$$ | 90.63 | 1.58 ± 0.48 ***##$$$ | 9.20 |

| | | | | | | |
|---|---|---|---|---|---|---|
| **Note: "***" indicates *P* < 0.001 compared to Group 1 (Vehicle); "##" indicates *P* < 0.01 compared to Group 2 (R-CHOP); "$$$" indicates *P* < 0.001 compared to Group 3.** | | | | | | |

Since animals in the Vehicle Group began to have TV > 2000 mm³ on Day 14 and were euthanized at the endpoint of the study, statistical analysis was performed based on the data from Day 14. Based on Table 6 and Figure 4, on Day 14, the mean TV of Group 4 (T-CHP) was 176.27 ± 63.80 mm³, the mean TV of Group 2 (R-CHOP) was 557.11 ± 76.43 mm³, the mean TV of Group 3 (TRS005) was 905.30 ± 129.66 mm³, and the mean TV of Group 4 (T-CHP) was significantly lower than that in Group 2 (R-CHOP) and Group 3 (TRS005). On Day 14, %TGI of Group 4, Group 3, and Group 2 were 90.63%, 51.87%, and 70.38%, respectively.

**Table 7 Changes in Tumor Volume of Mice in Each Treatment Group from D14 to D28**

| **Group** | **Number of Mice** | **Tumor Volume (mm³, Mean ± SEM)** | | | | |
|---|---|---|---|---|---|---|
| | | **Day 14** | **Day 17** | **Day 21** | **Day 24** | **Day 28** |
| 1 | 10 | 1881.01 ± 196.11 | 2329.47 ± 223.23 (N=6) | 2732.45 (N=1) | / | / |
| 2 | 10 | 557.11 ± 76.43 | 636.97 ± 90.22 | 1148.05 ± 140.62 | 1940.99 ± 225.03 | 1861.30 ± 773.20 (N=3) |
| 3 | 10 | 905.30 ± 129.66 | 1182.02 ± 162.95 | 1764.32 ± 236.31 (N=9) | 2274.64 ± 465.52 (N=6) | 0.00 (N=1) |
| 4 | 10 | 176.27 ± 63.80 ## | 177.68 ± 73.43 ## | 298.04 ± 139.63 ## | 515.41 ± 229.70 ## | 746.50 ± 228.09 (N=9) |

| | | | | | | |
|---|---|---|---|---|---|---|
| **"##" indicates *P* < 0.01 compared to Group 2 (R-CHOP)** | | | | | | |

TGI analysis was performed on D14. Since 4 mice in Group 1 had a TV > 2000 mm³ and were euthanized on D14, TGI analysis could not be performed from D14 to D28. Based on Table 7, the TV of mice in Group 4 (T-CHP) was significantly lower than that of Group 2 (R-CHOP) on D14 to D24, and the TGI followed a consistent trend from D0 to D14. On D24, 1 and 7 mice in Group 4 (T-CHP) and Group 2 (R-CHOP) had a TV> 2000 mm³, respectively, and were euthanized. By D28, only 3 mice in Group 2 (R-CHOP) had their TV measured, with TV of 327.42 mm³, 2458.12 mm³, and 2798.35 mm³, respectively, with a mean of 1861.30 ± 773.20; 9 mice in Group 4 (T-CHP) had their TV measured, with TV of 687.44 mm³, 241.60 mm³, 57.61 mm³, 281.22 mm³, 1243.65 mm³, 0.00 mm³, 723.03 mm³, 1810.62 mm³, and 1673.31 mm³, respectively, with a mean of 746.50 ± 228.09.

Based on the TV data of mice in each treatment group from D0 to D28, the TV ofmice in Group 4 (T-CHP) was significantly lower than that in Group 2 (R-CHOP) and Group 3 (TRS005), indicating that Group 4 (T-CHP) could significantly inhibit the growth of tumor cells with low CD20 expression, and the efficacy was significantly improved compared with Group 2 (R-CHOP) and Group 3 (TRS005).

### (II) Effect of Test Article on Body Weight of Tumor-bearing Animals

The results of the effect of TRS005 alone or in combination on the BW of OCI-LY19 tumor-bearing animals showed that on Day 14, the mean BW of animals in each group increased by 1.11% to 12.50% (0.24 g to 2.64 g) compared with the start of the study on Day 0. The effect of treatment on the BW of tumor-bearing animals in each group is shown in Table 8.

**Table 8 Effect of Treatment on Body Weight of Tumor-bearing Animals in Each Group**

| **Group** | **Number of Mice** | **Body Weight of Mice (g, Mean ± SEM)** | | **%BWC Day 14 vs Day 0** | **Number of Mice with Drug-related Deaths** |
|---|---|---|---|---|---|
| | | **Day 0** | **Day 14** | | |
| | | | | | **Day 14** |
| 1 | 10 | 21.27 ± 0.42 | 23.91 ± 0.40 | 12.50% (2.64 g) | 0/10 |
| 2 | 10 | 21.11 ± 0.26 | 21.78 ± 0.29 | 3.14% (0.67 g) | 0/10 |
| 3 | 10 | 20.95 ± 0.36 | 22.76 ± 0.45 | 8.96% (1.81 g) | 0/10 |
| 4 | 10 | 20.79±0.28 | 21.03±0.33 | 1.11% (0.24 g) | 0/10 |

During the study, the BWC of mice in each treatment group was within the normal range, no drug-related mouse deaths occurred, and no other obvious drug-related toxicity was observed, indicating that the drug combination regimen of TRS005 and CHP and the TRS005 monotherapy regimen of the present disclosure have good safety in inhibiting CD20 low-expressing tumor cells.

In summary, the present disclosure provides a new drug combination idea based on the existing first-line standard therapy for NHL, especially DLBCL. This approach modifies the current R-CHOP therapy by replacing Rituximab (R) and Vincristine (O) with the anti-CD20 ADC developed in this disclosure, thereby reducing the number of drugs used in the regimen. On the basis of reducing the number of medications, the drug combination regimen of the anti-CD20 ADC and CHP of the present disclosure has achieved a significantly improved tumor inhibition effect on DLBCL cells with both high and low CD20 expression compared with the R-CHOP regimen. In addition, the anti-CD20 ADC monotherapy of the present disclosure also achieved excellent efficacy on DLBCL cells with both high and low CD20 expression. It provides a clinical solution with fewer types of drugs, shorter infusion time, lower safety risks, and better economic accessibility, thereby presenting a superior option for the first-line treatment of DLBCL.

The above description of the detailed description of the present disclosure is not intended to limit the present disclosure. Those skilled in the art may make various changes or modifications according to the present disclosure, and such changes or modifications are intended to fall within the scope of the appended claims if they do not depart from the spirit of the disclosure.

## Claims

1. A use of an anti-CD20 antibody drug conjugate (ADC) alone or in combination with at least one therapeutic agent in the preparation of a drug for treating Non-Hodgkin Lymphoma (NHL), wherein the anti-CD20 ADC has the following structure: 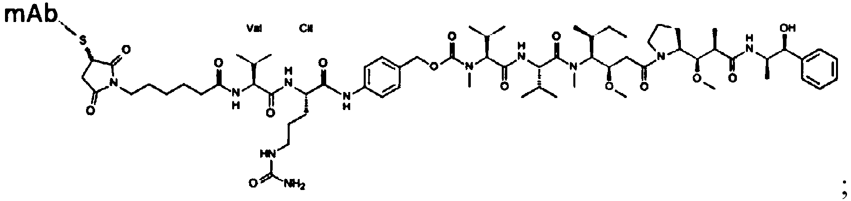
wherein mAb represents a recombinant anti-CD20 monoclonal antibody (mAb); preferably, the recombinant anti-CD20 mAb is Rituximab or a biosimilar thereof; the drug antibody ratio (DAR) is n, and n is 1 to 8; preferably, n is 4.2 ± 1; further preferably, n is 4.2 ± 0.5; and still more preferably, n is 4.2 ± 0.3;
wherein, the at least one therapeutic agent is selected from one or more of an alkylating agent, an antibiotic drug, a botanical drug, a hormone drug, a platinum drug, and an antimetabolite;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin;
the botanical drug is selected from one or more of Vinca Alkaloid, Vinblastine, Vincristine, Vindesine, Vinorelbine, Docetaxel, Camptothecin, Irinotecan, Exatecan, Topotecan, Paclitaxel, and Taxane;
the hormone drug is selected from one or more of Prednisone and Dexamethasone;
the platinum drug is selected from one or more of Carboplatin, Cisplatin, and Oxaliplatin; and
the antimetabolite is selected from one or more of Pemetrexed, Methotrexate, Fluorouracil, Capecitabine, Cytarabine, Gemcitabine, 6-Mercaptopurine, and Tioguanine.

2. The use according to claim 1, wherein the at least one therapeutic agent is an alkylating agent, an antibiotic drug, and a hormone drug;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin; and
the hormone drug is selected from one or more of Prednisone and Dexamethasone.

3. The use according to claim 2, wherein the at least one therapeutic agent is Cyclophosphamide, Doxorubicin, and Prednisone.

4. The use according to any one of claims 1 to 3, wherein the NHL is selected from small lymphocytic lymphoma (SLL), chronic lymphocytic leukemia (CLL), marginal zone lymphoma (MZL), follicular lymphoma (FL), diffuse large B-cell lymphoma (DLBCL), Burkitt lymphoma (BL), mantle cell lymphoma (MCL), Precursor B-cell lymphoblastic leukemia/lymphoma (B-ALL/LBL) , T-cell Large granular lymphocytic leukemia(T-LGLL), or anaplastic large cell lymphoma (ALCL).

5. The use according to claim 4, wherein the NHL is DLBCL; preferably, CD20-positive DLBCL.

6. The use according to claim 4, wherein the NHL is newly diagnosed DLBCL; preferably, newly diagnosed CD20-positive DLBCL.

7. The use according to claim 5, wherein the CD20-positive DLBCL is DLBCL with high or low CD20 expression.

8. The use according to claim 6, wherein the newly diagnosed CD20-positive DLBCL is newly diagnosed DLBCL with high or low CD20 expression.

9. The use according to claim 7, wherein the CD20-positive DLBCL is DLBCL with dual expression of MYC and BCL2, and high or low CD20 expression.

10. The use according to claim 8, wherein the newly diagnosed CD20-positive DLBCL is newly diagnosed DLBCL with dual expression of MYC and BCL2, and high or low CD20 expression.

11. A drug product for treating NHL, wherein the drug product comprises an anti-CD20 ADC and at least one therapeutic agent, and the anti-CD20 ADC has the following structure: 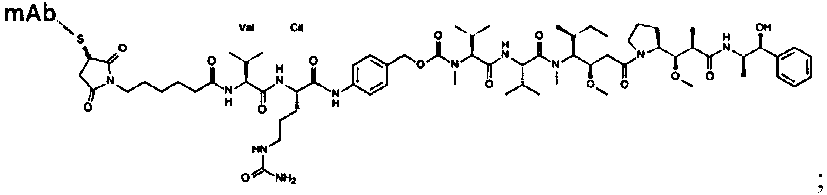
wherein mAb represents a recombinant anti-CD20 mAb; preferably, the recombinant anti-CD20 mAb is Rituximab or its biosimilar; the DAR is n, and n is 1 to 8; preferably, n is 4.2 ± 1; further preferably, n is 4.2 ± 0.5; and still more preferably, n is 4.2 ± 0.3;
wherein the at least one therapeutic agent is selected from one or more of an alkylating agent, an antibiotic drug, a botanical drug, a hormone drug, a platinum drug, and an antimetabolite;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin;
the botanical drug is selected from one or more of Vinca Alkaloid, Vinblastine, Vincristine, Vindesine, Vinorelbine, Docetaxel, Camptothecin, Irinotecan, Exatecan, Topotecan, Paclitaxel, and Taxane;
the hormone drug is selected from one or more of Prednisone and Dexamethasone;
the platinum drug is selected from one or more of Carboplatin, Cisplatin, and Oxaliplatin; and
the antimetabolite is selected from one or more of Pemetrexed, Methotrexate, Fluorouracil, Capecitabine, Cytarabine, Gemcitabine, 6-Mercaptopurine, and Tioguanine.

12. The drug product according to claim 11, wherein the at least one therapeutic agent is an alkylating agent, an antibiotic drug, and a hormone drug;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin; and
the hormone drug is selected from one or more of Prednisone and Dexamethasone;

13. The drug product according to claim 12, wherein the at least one therapeutic agent is Cyclophosphamide, Doxorubicin, and Prednisone.

14. The drug product according to any one of claims 11 to 13, wherein the NHL is DLBCL; preferably, CD20-positive DLBCL.

15. The drug product according to claim 14, wherein the NHL is newly diagnosed DLBCL; preferably, newly diagnosed CD20-positive DLBCL.

16. The drug product according to claim 15, wherein the newly diagnosed CD20-positive DLBCL is newly diagnosed DLBCL with high or low CD20 expression.

17. The drug product according to claim 16, wherein the newly diagnosed CD20-positive DLBCL is newly diagnosed DLBCL with dual expression of MYC and BCL2, and high or low CD20 expression.

18. A method for treating NHL, wherein an effective dose of an anti-CD20 ADC or a combination thereof with at least one therapeutic agent is administered to a subject, and the anti-CD20 ADC has the following structure: 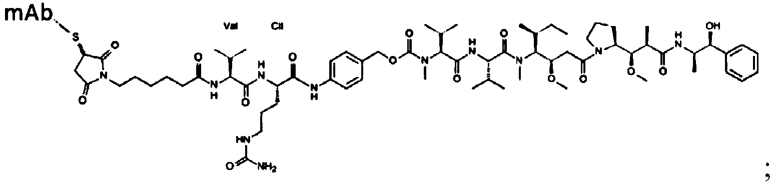
wherein mAb represents a recombinant anti-CD20 mAb; preferably, the recombinant anti-CD20 mAb is Rituximab or its biosimilar; the DAR is n, and n is 1 to 8; preferably, n is 4.2 ± 1; further preferably, n is 4.2 ± 0.5; and still more preferably, n is 4.2 ± 0.3;
wherein the at least one therapeutic agent is selected from one or more of an alkylating agent, an antibiotic drug, a botanical drug, a hormone drug, a platinum drug, and an antimetabolite;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin;
the botanical drug is selected from one or more of Vinca Alkaloid, Vinblastine, Vincristine, Vindesine, Vinorelbine, Docetaxel, Camptothecin, Irinotecan, Exatecan, Topotecan, Paclitaxel, and Taxane;
the hormone drug is selected from one or more of Prednisone and Dexamethasone;
the platinum drug is selected from one or more of Carboplatin, Cisplatin, and Oxaliplatin; and
the antimetabolite is selected from one or more of Pemetrexed, Methotrexate, Fluorouracil, Capecitabine, Cytarabine, Gemcitabine, 6-Mercaptopurine, and Tioguanine.

19. The treatment method according to claim 18, wherein the at least one therapeutic agent is an alkylating agent, an antibiotic drug, and a hormone drug;
the alkylating agent is selected from one or more of Cyclophosphamide, Ifosfamide, Melphalan, Busulfan, Chlormethine, Chlorambucil, Thiotepa, Bendamustine, Carmustine, Nimustine, Lomustine, and Semustine;
the antibiotic drug is selected from one or more of Doxorubicin, Daunorubicin, Bleomycin, Mitomycin, Epirubicin, Aclarubicin, and Idarubicin; and
the hormone drug is selected from one or more of Prednisone and Dexamethasone;

20. The treatment method according to claim 19, wherein the at least one therapeutic agent is Cyclophosphamide, Doxorubicin, and Prednisone.

21. The treatment method according to any one of claims 18 to 20, wherein the NHL is DLBCL; preferably, CD20-positive DLBCL.

22. The treatment method according to claim 21, wherein the NHL is newly diagnosed DLBCL; preferably, newly diagnosed CD20-positive DLBCL.

23. The treatment method according to claim 22, wherein the newly diagnosed CD20-positive DLBCL is newly diagnosed DLBCL with high or low CD20 expression.

24. The treatment method according to claim 23, wherein the newly diagnosed CD20-positive DLBCL is newly diagnosed DLBCL with dual expression of MYC and BCL2, and high or low CD20 expression.

25. The treatment method according to any one of claims 18 to 24, wherein the anti-CD20 ADC can be administered before, simultaneously with, and/or after the administration of the at least one therapeutic agent.

26. A method for non-therapeutic inhibition of NHL cells, wherein an effective dose of the drug product as described in any one of claims 11 to 17 is added to the NHL cell system.
